# EUROPÄISCHE PATENTANMELDUNG

(11) **EP 4 772 592 A1**
(43) Veröffentlichungstag der Anmeldung: **08.07.2026**
(21) Anmeldenummer: 25150288.6
(22) Anmeldetag: 06.01.2025
(51) Int. Cl.: C09J 175/00, C08G 18/48

(54) **HÄRTBARE ZUSAMMENSETZUNG MIT ALDIMIN-FUNKTIONELLEM POLYMER UND 1,3-KETOESTER**

(71) Anmelder: Sika Technology AG, 6340 Baar (CH)
(72) Erfinder: KRAMER, Andreas, 8048 Zürich (CH); BURCKHARDT, Urs, 8048 Zürich (CH); HÄBERLE, Hans, 8048 Zürich (CH); STADELMANN, Ursula, 8048 Zürich (CH)
(74) Vertreter: Sika Patent Attorneys

(57) **Zusammenfassung**

Gegenstand der Erfindung ist eine härtbare Zusammensetzung umfassend mindestens ein Polymer mit Endgruppen der Formel (I) enthaltend eine aromatische Aldimingruppe und einem mittleren Molekulargewicht Mₙ von 1'000 bis 30'000 g/mol, und mindestens eine Verbindung mit zwei oder mehr 1,3-Ketoestergruppen der Formel (II).

Die härtbare Zusammensetzung ist nicht empfindlich auf Feuchtigkeit, härtet bei Raumtemperatur beim Vermischen der Komponenten schnell und zuverlässig aus und ermöglicht eine überraschend hohe Dehnbarkeit und einen überraschend hohen Weiterreisswiderstand bei hoher Festigkeit und guter Stabilität gegenüber Hitze und Hydrolyse.

## Beschreibung

### Technisches Gebiet

Die Erfindung betrifft bei Raumtemperatur härtbare elastische Kleb- oder Dichtstoffe mit Aldimin-funktionellem Polymer und 1,3-Ketoester.

### Stand der Technik

Reaktive Polymerzusammensetzungen, die bei Raumtemperatur aushärten und als Klebstoffe, Dichtstoffe oder Beschichtungen mit elastischen Eigenschaften verwendet werden können, sind bekannt. Weit verbreitet sind Polyurethansysteme, welche durch die Reaktion von Isocyanatgruppen mit Polyolen und/oder Feuchtigkeit aushärten und besonders hochelastische Polymere bilden. Die Herstellung und Verwendung von Polyurethansystemen stellt in der Praxis aber eine Reihe von Herausforderungen, insbesondere aufgrund der Toxizität der enthaltenen monomeren Isocyanate und der hohen Empfindlichkeit gegenüber Feuchtigkeit vor und während der Aushärtung.

US 2018/0327535 beschreibt Aldimin-funktionelle Polymere und deren Aushärtung mittels Polyisocyanat unter dem Einfluss von Feuchtigkeit. Dabei bestehen die erwähnten Probleme mit toxischen Monomeren und Empfindlichkeit gegenüber Feuchtigkeit aber weiterhin.

WO 2024/012828 beschreibt Aldehyd-funktionelle Polymere und deren Aushärtung mit 1,3-Ketoestern. Die ausgehärteten Polymere sind in Bezug auf Dehnbarkeit und Weiterreisswiderstand noch verbesserungsfähig.

US 5,288,804 beschreibt lösemittelbasierte Lacke auf der Basis von Acetoacetat-funktionellen Acrylatpolymeren, welche mit aromatischen Polyaldiminen aushärten. Dabei entstehen hohe Emissionen, und die erhaltenen Lacke sind nicht elastisch und kaum dehnbar.

US 5,536,784 beschreibt wässrige Beschichtungen aus der Kombination von Acetoacetat-funktionellen und Aldimin-funktionellen Polyesterurethandispersionen. Solche wässrigen Systeme sind in hoher Schichtdicke oder zwischen wasserundurchlässigen Substraten nicht einsetzbar, und die erhaltenen Filme sind nicht elastisch.

### Darstellung der Erfindung

Aufgabe der vorliegenden Erfindung ist es, eine bei Raumtemperatur härtbare Zusammensetzung zur Verfügung zu stellen, welche eine hohe Dehnbarkeit aufweist, geeignet ist als elastischer Kleb- oder Dichtstoff und die Nachteile des Standes der Technik in Bezug auf toxische Monomere und Empfindlichkeit gegenüber Feuchtigkeit vor und während der Aushärtung überwindet. Überraschenderweise wird diese Aufgabe mit einer härtbaren Zusammensetzung wie in Anspruch 1 beschrieben gelöst. Die Zusammensetzung umfasst ein Polymer mit Endgruppen der Formel (I) und eine Verbindung mit zwei oder mehr 1,3-Ketoestergruppen der Formel (II). Die Endgruppe der Formel (I) enthält eine aromatische Aldimingruppe und ist über eine Urethan-, Thiourethan- oder Harnstoffgruppe, insbesondere über eine Urethangruppe, ans Polymer gebunden.

Die erfindungsgemässe Zusammensetzung enthält keine toxischen Monomere und ist nicht empfindlich auf Feuchtigkeit. Ihre Komponenten lassen sich einfach herstellen und ohne besondere Vorkehrungen lange lagern. Nach dem Vermischen der Komponenten härtet die Zusammensetzung bei Raumtemperatur schnell und geruchlos aus, ohne dafür zugesetzte Katalysatoren zu benötigen. Die ausgehärtete Zusammensetzung zeigt eine überraschend hohe Dehnbarkeit und Elastizität bei hoher Festigkeit und hohem Weiterreisswiderstand, ausserdem eine gute Stabilität gegenüber Hitze und Hydrolyse.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine härtbare Zusammensetzung umfassend
- mindestens ein Polymer mit Endgruppen der Formel (I) und einem mittleren Molekulargewicht Mₙ von 1'000 bis 30'000 g/mol, 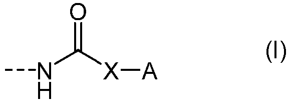 wobei
   X für O, S oder NR¹ und R¹ für H oder einen einwertigen Kohlenwasserstoffrest mit 1 bis 18 C-Atomen steht, und
   A für einen einwertigen organischen Rest mit 7 bis 30 C-Atomen steht, der eine aromatische Aldimingruppe enthält,
und
- mindestens eine Verbindung mit zwei oder mehr 1 ,3-Ketoestergruppen der Formel (II), 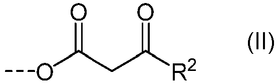 wobei R² für einen einwertigen Kohlenwasserstoffrest mit 1 bis 6 C-Atomen steht.

Eine gestrichelte Linie in den Formeln in diesem Dokument stellt jeweils die Bindung zwischen einem Substituenten und dem zugehörigen Molekülrest dar.

Als "aromatische Aldimingruppe" wird eine Gruppierung der Formel --N=CH-bezeichnet, deren Kohlenstoffatom direkt an einen aromatischen oder heteroaromatischen Ring gebunden ist und die selbst nicht Teil eines heteroaromatischen Ringsystems ist.

Als "Molekulargewicht" wird die molare Masse (in Gramm pro Mol) eines Moleküls bezeichnet. Als "mittleres Molekulargewicht" wird das Zahlenmittel des Molekulargewichts Mₙ einer polydispersen Mischung von oligomeren oder polymeren Molekülen bezeichnet. Es wird mittels Gelpermeationschromatographie (GPC) gegen Polystyrol als Standard bestimmt.

Mit "Poly" beginnende Substanznamen wie Polyol oder Polyaldimin bezeichnen Substanzen, die formal zwei oder mehr der in ihrem Namen vorkommenden funktionellen Gruppen pro Molekül enthalten.

Als "lagerstabil" wird eine Zusammensetzung bezeichnet, wenn sie bei Raumtemperatur in einem geeigneten Gebinde während längerer Zeit, typischerweise während mindestens drei Monaten bis zu sechs Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "Raumtemperatur" wird eine Temperatur von 23 °C bezeichnet.

Alle im Dokument erwähnten Industriestandards und Normen beziehen sich auf die zum Zeitpunkt der Einreichung der Erstanmeldung gültigen Fassungen. Gewichtsprozente (Gewichts-%) bezeichnen Massenanteile eines Bestandteils einer Zusammensetzung oder eines Moleküls, bezogen auf die gesamte Zusammensetzung oder das gesamte Molekül, falls nichts anderes angeben ist. Die Begriffe "Masse" und "Gewicht" werden im vorliegenden Dokument synonym benutzt.

Bevorzugt ist das Polymer mit Endgruppen der Formel (I) bei Raumtemperatur flüssig. Insbesondere hat es eine Viskosität bei 20 °C von 1 bis 500 Pa s, besonders bevorzugt 2 bis 200 Pa s, insbesondere 3 bis 150 Pa s, gemessen mittels Kegel-Platten Viskosimeter mit Kegeldurchmesser 10 mm, Kegelwinkel 1°, Kegelspitze-Platten-Abstand 0.05 mm, Scherrate 10 s⁻¹.

Bevorzugt weist das Polymer mit Endgruppen der Formel (I) ein mittleres Molekulargewicht Mₙ von 2'000 bis 20'000 g/mol, insbesondere 2'500 bis 15'000 g/mol, auf.

Bevorzugt weist das Polymer mit Endgruppen der Formel (I) eine mittlere Aldimin-Funktionalität von 1.5 bis 4, bevorzugt 1.8 bis 3, insbesondere 2 bis 3, auf.

Besonders bevorzugt weist das Polymer mit Endgruppen der Formel (I) ein mittleres Molekulargewicht Mₙ von 2'000 bis 20'000 g/mol, bevorzugt 2'500 bis 15'000 g/mol, und eine mittlere Aldimin-Funktionalität von 1.8 bis 3, bevorzugt 2 bis 3, auf.

Bevorzugt weist das Polymer mit Endgruppen der Formel (I) ein Polyether- oder ein Polyester-Rückgrat auf.

Besonders bevorzugt weist das Polymer mit Endgruppen der Formel (I) ein Polyether-Rückgrat auf.

Insbesondere enthält das Polyether-Rückgrat Repetiereinheiten ausgewählt aus der Liste bestehend aus Oxy-1,2-propylen, Oxy-1,3-propylen, Oxy-1,4-butylen, Oxy-1,2-butylen und Oxyphenylethylen. Zusätzlich kann das Polyether-Rückgrat einen Anteil von bis zu 25 Gewichts-% Oxy-1,2-ethylen-Einheiten bezogen auf das gesamte Polyether-Rückgrat enthalten.

Am meisten bevorzugt ist ein Poly(oxy-1,2-propylen)-Rückgrat, welches bis zu 25 Gewichts-%, bevorzugt bis zu 20 Gewichts-%, Oxy-1,2-ethylen-Einheiten bezogen auf das gesamte Polyether-Rückgrat enthalten kann, insbesondere an den Kettenenden.

Ein Polymer mit Endgruppen der Formel (I) mit einem Polyether-Rückgrat weist bevorzugt ein mittleres Molekulargewicht Mₙ von 4'000 bis 15'000 g/mol auf.

Das Polymer enthält Endgruppen der Formel (I), 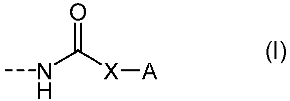 wobei
X für O, S oder NR¹ und R¹ für H oder einen einwertigen Kohlenwasserstoffrest mit 1 bis 18 C-Atomen steht, und
A für einen einwertigen organischen Rest mit 7 bis 30 C-Atomen steht, der eine aromatische Aldimingruppe enthält.

Bevorzugt steht R¹ für Methyl, Ethyl, Cyclohexyl oder Benzyl.

Bevorzugt steht X für O. Eine solche Endgruppe der Formel (I) ist über eine Urethangruppe ans Polymer gebunden. Ein solches Polymer ist besonders einfach zugänglich und ermöglicht eine besonders hohe Dehnbarkeit.

In den Endgruppen der Formel (I) steht A bevorzugt für einen Rest der Formel

- - - G¹-N=CH-Z

oder

- - - G²-CH=N-R³, wobei

G¹ für einen zweiwertigen aliphatischen, cycloaliphatischen oder arylaliphatischen, gegebenenfalls Ether-Sauerstoff aufweisenden Kohlenwasserstoff-Rest mit 2 bis 18 C-Atomen steht,
Z für einen aromatischen oder heteroaromatischen fünf- oder sechsgliedrigen Ring steht, der gegebenenfalls substituiert und/oder anelliert ist und insgesamt 4 bis 25 C-Atome umfasst,
G² für einen zweiwertigen, gegebenenfalls Sauerstoffatome enthaltenden arylaliphatischen Kohlenwasserstoffrest mit 5 bis 12 C-Atomen steht, welcher über ein aliphatisches C-Atom an X und über einen aromatischen oder heteroaromatischen Ring an CH=N gebunden ist, und
R³ für einen einwertigen, gegebenenfalls Ether-Sauerstoff enthaltenden aliphatischen, cycloaliphatischen oder arylaliphatischen Kohlenwasserstoffrest mit 1 bis 13 C-Atomen steht.

Bevorzugt stehen die Endgruppen der Formel (I) somit für Endgruppen der Formel (la) oder für Endgruppen der Formel (Ib), 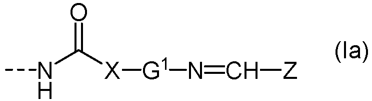 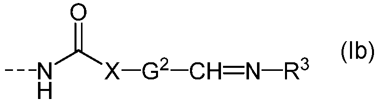 wobei X, G¹, Z, G² und R³ die bereits genannten Bedeutungen aufweisen.

Bevorzugt steht Z für einen Phenylrest, einen Naphthylrest, einen Furylrest, einen Pyridinrest, einen Pyrrolrest, einen Indolrest oder einen Thiophenrest, insbesondere einen Phenylrest, Naphthylrest oder Furylrest, wobei diese Reste gegebenenfalls substituiert sind, insbesondere mit Alkyl- oder Alkoxy- oder Nitro- oder Estergruppen.

Insbesondere ist Z ausgewählt aus der Liste bestehend aus Phenyl, 2-Methylphenyl, 3-Methylphenyl, 4-Methylphenyl, 4-Ethylphenyl, 4-Isopropylphenyl, 4-tert-Butylphenyl, 4-C₁₀₋₁₄-Alkylphenyl, 2,5-Dimethylphenyl, 2-Methoxyphenyl, 3-Methoxyphenyl, 4-Methoxyphenyl, 2-Ethoxyphenyl, 3-Ethoxyphenyl, 4-Ethoxyphenyl, 4-Propoxyphenyl, 4-Isopropoxyphenyl, 4-Butoxyphenyl, 4-Pentoxyphenyl, 4-Decyloxyphenyl, 4-Dodecyloxyphenyl, 2,3-Dimethoxyphenyl, 2,4-Dimethoxyphenyl, 2,5-Dimethoxyphenyl, 3,4-Dimethoxyphenyl, 3,5-Dimethoxyphenyl, 2,4,6-Trimethylphenyl, 2,4,5-Trimethoxyphenyl, 2,4,6-Trimethoxyphenyl, 3,4,5-Trimethoxyphenyl, 4-Methoxycarbonylphenyl, 4-Ethoxycarbonylphenyl, 3-Nitrophenyl, 1-Naphthyl, 2-Naphthyl, 2-Furyl und 5-Methyl-2-furyl.

Davon bevorzugt ist Phenyl, 4-Methoxycarbonylphenyl, 4-Ethoxycarbonylphenyl, 3-Nitrophenyl, 2-Furyl oder 5-Methyl-2-furyl.

Besonders bevorzugt steht Z für Phenyl. Solche Endgruppen der Formel (I) sind abgeleitet von Benzaldehyd. Sie sind technisch wie kommerziell besonders gut zugänglich und ermöglichen eine schnelle Aushärtung.

Weiterhin besonders bevorzugt steht Z für 2-Furyl oder 5-Methyl-2-furyl. Solche Aldimingruppen ermöglichen eine besonders schnelle Aushärtung.

Aldimine abgeleitet von Benzaldehyd oder anderen flüchtigen Aldehyden verursachen in härtbaren Polyurethan-Zusammensetzungen typischerweise einen starken, unangenehmen Geruch, welcher während und nach der Applikation deutlich wahrnehmbar ist und ihre Verwendung stark einschränkt. Im Fall der erfindungsgemässen härtbaren Zusammensetzung entsteht aber kein Aldehyd-Geruch, was besonders vorteilhaft ist, da die Zusammensetzung ohne Einschränkung auch in geruchssensitiven Anwendungen eingesetzt werden kann, beispielsweise in Gebäudeinnenräumen oder in Automobilen.

Bevorzugt steht G¹ für einen zweiwertigen aliphatischen, gegebenenfalls Ether-Sauerstoff aufweisenden Kohlenwasserstoff-Rest mit 4 bis 12 C-Atomen. Insbesondere ist G¹ ausgewählt aus der Gruppe bestehend aus 1,2-Ethylen, 1,2-Propylen, 1,3-Propylen, 1,4-Butylen, 1,5-Pentylen, 1,6-Hexylen, 1,8-Octylen, 1,10-Decylen, 1,12-Dodecylen, (1,5,5-Trimethylcyclohexan-1-yl)methan-1,3 , 3-Oxa-1,5-pentylen und 3, 6-Dioxa-1,8-octylen.

Besonders bevorzugt steht G¹ für 3-Oxa-1,5-pentylen.

Bevorzugt steht G² für einen Rest der Formel

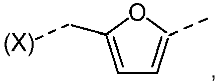

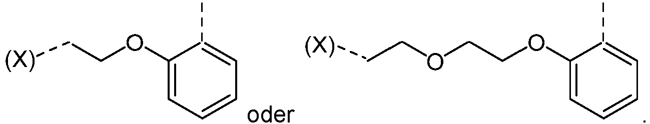

Besonders bevorzugt steht G² für einen Rest der Formel

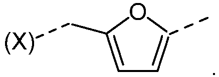

Bevorzugt steht R³ für einen Rest ausgewählt aus der Liste bestehend aus Methyl, Ethyl, Propyl, Isopropyl, Butyl, Isobutyl, tert.Butyl, 2-Butyl, Pentyl, 2-Pentyl, 3-Methylbutyl, 3-Methyl-2-butyl, Hexyl, Octyl, 2-Ethylhexyl, Nonyl, Decyl, Tridecyl, 2-Methoxyethyl, 2-Ethoxyethyl, 3-Methoxypropyl, 3-Ethoxypropyl, Cyclohexyl, Benzyl oder 2-Phenylethyl.

Davon bevorzugt ist Butyl, Hexyl, Octyl, 2-Ethylhexyl, 2-Methoxyethyl, 2-Ethoxyethyl, 3-Methoxypropyl, 3-Ethoxypropyl oder Benzyl.

Besonders bevorzugt ist Butyl oder Benzyl.

Bevorzugt wird das Polymer mit Endgruppen der Formel (I) erhalten durch die Umsetzung eines Isocyanatgruppen-haltigen Polymers mit einem Aldimin der Formel HX-A in einem Verhältnis der Anzahl Mol Isocyanatgruppen zur Anzahl Mol Aldimin der Formel HX-A von mindestens 1, wobei A und X die bereits genannten Bedeutungen aufweisen.

Besonders bevorzugt wird das Polymer mit Endgruppen der Formel (I) erhalten durch die Umsetzung eines Isocyanatgruppen-haltigen Polymers mit einem Aldimin der Formel (Illa) oder der Formel (IIb) in einem Verhältnis der Anzahl Mol Isocyanatgruppen zur Anzahl Mol Aldimin der Formel (Illa) oder (IIIb) von mindestens 1,

HX-G¹-N=CH-Z (IIIa)

HX-G²-CH=N-R³ (IIb)

wobei X, G¹, G², Z und R³ die bereits genannten Bedeutungen aufweisen.

In einer bevorzugten Ausführungsform der Erfindung sind die Endgruppen der Formel (I) Endgruppen der Formel (la). Ein solches Polymer wird insbesondere erhalten durch die Umsetzung eines Isocyanatgruppen-haltigen Polymers mit einem Aldimin der Formel (IIIa) in einem Verhältnis der Anzahl Mol Isocyanatgruppen zur Anzahl Mol Aldimin der Formel (Illa) von mindestens 1, insbesondere in einem Verhältnis von 1.0 bis 1.2, bevorzugt 1.0 bis 1.1.

Ein geeignetes Aldimin der Formel (Illa) wird bevorzugt erhalten durch die Umsetzung eines Amins der Formel HX-G¹-NH₂ mit einem Aldehyd der Formel O=CH-Z unter Freisetzung von Wasser, insbesondere in einem stöchiometrischen Verhältnis von etwa 1/1. Bevorzugt werden das Amin und der Aldehyd vermischt und das freigesetzte Wasser mittels Vakuumdestillation entfernt.

Als Amin der Formel HX-G¹-NH₂ geeignet ist insbesondere N-Methyl-1,2-ethan-diamin, N-Methyl-1,2-propandiamin, N-Methyl-1,3-propandiamin, N-Methyl-1,4-butandiamin, N-Methyl-1,5-pentandiamin, N-Methyl-1,6-hexandiamin, N-Methyl-1,8-octandiamin, N-Methyl-1,10-decandiamin, N-Methyl-1,12-dodecandiamin, die genannten Amine mit Ethyl oder Cyclohexyl oder Benzyl anstelle von Methyl am Stickstoff, sowie weiterhin 5-Aminopentanol, 6-Aminohexanol, 8-Aminooctanol, 10-Aminodecanol, 12-Aminododecanol, 3-Aminomethyl-3,5,5-trimethylcyclohexanol, 2-(2-Aminoethoxy)ethanol oder 2-(2-(2-Aminoethoxy)ethoxy)ethanol, sowie weiterhin Additionsprodukte von primären Diaminen an Maleinsäurediester im Molverhältnis 1:1, wie insbesondere das Additionsprodukt von Isophorondiamin an Maleinsäuredimethylester oder Maleinsäurediethylester im Molverhältnis 1:1.

Besonders bevorzugt als Amin der Formel HX-G¹-NH₂ ist 2-(2-Aminoethoxy)-ethanol.

Als Aldehyd der Formel O=CH-Z geeignet ist insbesondere Benzaldehyd, 2-Methylbenzaldehyd, 3-Methylbenzaldehyd, 4-Methylbenzaldehyd, 4-Ethylbenzaldehyd, 4-Isopropylbenzaldehyd, 4-tert-Butylbenzaldehyd, 4-C₁₀₋₁₄-Alkylbenzaldehyd, 2,5-Dimethylbenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Propoxybenzaldehyd, 4-Isopropoxybenzaldehyd, 4-Butoxybenzaldehyd, 4-Pentoxybenzaldehyd, 4-Decyloxybenzaldehyd, 4-Dodecyloxybenzaldehyd, 2,3-Dimethoxybenzaldehyd, 2,4-Dimethoxybenzaldehyd, 2,5-Dimethoxybenzaldehyd, 3,4-Dimethoxybenzaldehyd, 3,5-Dimethoxybenzaldehyd, 2,4,6-Trimethylbenzaldehyd, 2,4,5-Trimethoxybenzaldehyd, 2,4,6-Trimethoxybenzaldehyd, 3,4,5-Trimethoxybenzaldehyd, 4-Formylbenzoesäureester, insbesondere 4-Formylbenzoesäuremethylester oder 4-Formylbenzoesäureethylester, 3-Nitrobenzaldehyd, 1-Naphthaldehyd, 2-Naphthaldehyd, Furfural oder 5-Methylfurfural.

Bevorzugt sind Benzaldehyd, 4-Formylbenzoesäuremethylester, 4-Formylbenzoesäureethylester, 3-Nitrobenzaldehyd, 2-Methylbenzaldehyd, 3-Methylbenzaldehyd, 4-Methylbenzaldehyd, 4-Methoxybenzaldehyd, 3,4-Dimethoxybenzaldehyd, Furfural oder 5-Methylfurfural, insbesondere Benzaldehyd, 4-Formylbenzoesäuremethylester, 3-Nitrobenzaldehyd, Furfural oder 5-Methylfurfural.

Das Aldimin der Formel (Illa) ist insbesondere ausgewählt aus der Liste bestehend aus N-Benzyliden-5-aminopentanol, N-Benzyliden-6-aminohexanol, N-Benzyliden-8-aminooctanol, N-Benzyliden-10-aminodecanol, N-Benzyliden-12-aminododecanol, N-Benzyliden-3-aminomethyl-3,5,5-trimethylcyclohexanol, N-Benzyliden-2-(2-aminoethoxy)ethanol, N-Benzyliden-2-(2-(2-aminoethoxy)ethoxy)ethanol und entsprechenden Verbindungen abgeleitet von 4-Formylbenzoesäuremethylester, 4-Formylbenzoesäureethylester, 3-Nitrobenzaldehyd, Furfural und 5-Methylfurfural anstelle von Benzaldehyd.

Besonders geeignet ist N-Benzyliden-2-(2-aminoethoxy)ethanol, N-(4-Methoxycarbonyl)benzyliden-2-(2-aminoethoxy)ethanol, N-(4-Ethoxycarbonyl)benzyliden-2-(2-aminoethoxy)ethanol, N-(3-Nitro)benzyliden-2-(2-aminoethoxy)ethanol, N-Furfuryliden-2-(2-aminoethoxy)ethanol oder N-(5-Methylfurfuryliden)-2-(2-aminoethoxy)-ethanol.

In einer weiteren bevorzugten Ausführungsform der Erfindung sind die Endgruppen der Formel (I) Endgruppen der Formel (Ib). Ein solches Polymer wird insbesondere erhalten durch die Umsetzung eines Isocyanatgruppen-haltigen Polymers mit einem Aldimin der Formel (IIb) in einem Verhältnis der Anzahl Mol Isocyanatgruppen zur Anzahl Mol Aldimin der Formel (IIb) von mindestens 1, insbesondere von 1.0 bis 1.2, bevorzugt 1.0 bis 1.1.

Ein Aldimin der Formel (IIIb) wird seinerseits erhalten durch die Umsetzung eines Aldehyds der Formel (IV) mit einem Amin der Formel R³-NH₂ unter Freisetzung von Wasser, insbesondere in einem stöchiometrischen Verhältnis von etwa 1/1. Bevorzugt werden das Amin und der Aldehyd vermischt und das freigesetzte Wasser mittels Vakuumdestillation entfernt.

HX-G²-CH=O (IV)

Als Aldehyd der Formel (IV) geeignet ist insbesondere 5-Hydroxymethylfurfural oder ethoxylierter Salicylaldehyd, insbesondere 2-(2-Hydroxyethoxy)benzaldehyd und/oder 2-(2-(2-Hydroxyethoxy)ethoxy)benzaldehyd.

Als Aldimin der Formel (IIIb) geeignet ist insbesondere N-(5-Hydroxymethylfurfuryliden)butanamin, N-(5-Hydroxymethylfurfuryliden)benzylamin, N-(2-(2-hydroxyethoxy)benzyliden)butanamin und/oder N-(2-(2-(2-hydroxyethoxy)-ethoxy)benzyliden)butanamin, N-(2-(2-hydroxyethoxy)benzyliden)benzylamin und/oder N-(2-(2-(2-hydroxyethoxy)ethoxy)benzyliden)benzylamin, sowie entsprechende Verbindungen mit weiteren der bereits genannten Resten R³ anstelle von Butyl oder Benzyl.

Anstatt mit einem Aldimin der Formel (IIIb) kann die Umsetzung des Isocyanatgruppen-haltigen Polymers auch zuerst mit dem Aldehyd der Formel (IV) in einem Verhältnis der Anzahl Mol Isocyanatgruppen zur Anzahl Mol Aldehyd der Formel (IV) von mindestens 1 erfolgen, insbesondere in einem Verhältnis von 1.0 bis 1.2, bevorzugt 1.0 bis 1.1, gefolgt von der Umsetzung des erhaltenen Aldehyd-funktionellen Polymers mit einem Amin der Formel R³-NH₂ unter Freisetzung von Wasser, insbesondere in einem Verhältnis der Anzahl Mol Aldehydgruppen zur Anzahl Mol Amin der Formel R³-NH₂ von etwa 1.

Die Umsetzung des Isocyanatgruppen-haltigen Polymers mit dem Aldimin der Formel (Illa) oder (IIIb) erfolgt bevorzugt bei einer Temperatur von 20 bis 140 °C, bis alle Isocyanatgruppen umgesetzt sind, gegebenenfalls unter Mitverwendung eines geeigneten Katalysators. Im Fall von X = O erfolgt die Umsetzung bevorzugt bei einer Temperatur von 40 bis 140 °C, insbesondere 60 bis 120 °C, gegebenenfalls in Anwesenheit eines geeigneten Katalysators.

Als Isocyanatgruppen-haltiges Polymer geeignet ist insbesondere ein Umsetzungsprodukt von mindestens einem monomeren Diisocyanat mit mindestens einem polymeren Polyol in einem NCO/OH-Verhältnis von mindestens 1.5/1, bevorzugt mindestens 1.8/1.

Besonders bevorzugt ist ein Umsetzungsprodukt von mindestens einem monomeren Diisocyanat mit mindestens einem polymeren Polyol in einem NCO/OH-Verhältnis von 3/1 bis 10/1, bevorzugt 4/1 bis 9/1, insbesondere 5/1 bis 8/1, und nachfolgender Entfernung des monomeren Diisocyanats mittels eines geeigneten Trennverfahrens bis auf einen Gehalt von weniger als 0.5 Gewichts-%, bevorzugt weniger als 0.2 Gewichts-%, insbesondere weniger als 0.1 Gewichts-%, bezogen auf das Polymer.

Als monomeres Diisocyanat geeignet ist insbesondere 1,5-Pentandiisocyanat (PDI), 1,6-Hexandiisocyanat (HDI), 2,2(4),4-Trimethyl-1,6-hexandiisocyanat (TMDI), 1-Methyl-2,4(6)-diisocyanatocyclohexan (H₆TDI), Isophorondiisocyanat (IPDI), 4,4'-Diisocyanatodicyclohexylmethan (H₁₂MDI), 4(2),4'-Diphenylmethandiisocyanat (MDI) oder 2,4(6)-Toluendiisocyanat (TDI). Bevorzugt sind HDI oder IPDI, besonders bevorzugt ist IPDI.

Als polymeres Polyol geeignet sind insbesondere Polymere mit einer OH-Zahl von 9 bis 114 mg KOH/g, bevorzugt 12 bis 57 mg KOH/g, insbesondere 18 bis 45 mg KOH/g.

Als polymeres Polyol geeignet sind insbesondere Polyetherpolyole oder Polyesterpolyole.

Besonders bevorzugt sind Polyetherpolyole, auch Poly(oxyalkylen)polyole genannt.

Bevorzugte Polyetherpolyole sind Polymerisationsprodukte von Ethylenoxid oder 1,2-Propylenoxid oder 1,2- oder 2,3-Butylenoxid oder Oxetan oder Tetrahydrofuran, oder Mischungen davon, wobei diese mit Hilfe eines Startermoleküls mit zwei oder mehreren aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen.

Besonders bevorzugt sind Poly(oxy-1,2-propylen)diole, Poly(oxy-1,2-propylen)-triole oder sogenannte Ethylenoxid-terminierte (EO-endcapped oder EO-tipped) Poly(oxy-1,2-propylen)diole oder -triole. Letztere sind Polyoxyethylen-polyoxypropylen-Mischpolyole, die insbesondere dadurch erhalten werden, dass Polyoxypropylendiole oder -triole nach Abschluss der Propoxylierungsreaktion mit Ethylenoxid weiter alkoxyliert werden und dadurch schliesslich primäre Hydroxylgruppen aufweisen.

Weiterhin besonders bevorzugt sind Poly(oxy-1,3-propylen)diole oder Poly(oxy-1,4-butylen)diole.

Bevorzugt sind Polyole mit einem Ungesättigtheitsgrad von weniger als 0.02 meq/g, insbesondere weniger als 0.01 meq/g.

Besonders bevorzugt ist das Isocyanatgruppen-haltige Polymer ein Umsetzungsprodukt von einem Ethylenoxid-terminierten Poly(oxy-1,2-propylen)triol mit einer OH-Zahl von 28 bis 34 mg KOH/g und IPDI in einem NCO/OH-Verhältnis von 3/1 bis 10/1 und nachfolgender Entfernung von monomerem IPDI.

Das Isocyanatgruppen-haltige Polymer hat bevorzugt eine mittlere NCO-Funktionalität von 1.8 bis 3, bevorzugt 2 bis 3.

Das Isocyanatgruppen-haltige Polymer hat bevorzugt ein mittleres Molekulargewicht Mₙ von 1'200 bis 20'000 g/mol, bevorzugt 2'200 bis 15'000 g/mol, insbesondere 4'000 bis 10'000 g/mol.

Das Isocyanatgruppen-haltige Polymer hat bevorzugt einen NCO-Gehalt bezogen auf das Isocyanatgruppen-haltige Polymer von 0.6 bis 6 Gewichts-%, bevorzugt 0.9 bis 3.5 Gewichts-%, insbesondere 1.3 bis 2.7 Gewichts-%.

Der NCO-Gehalt wird insbesondere bestimmt durch Umsetzung der Isocyanatgruppen mit einem Überschuss Dibutylamin und Rücktitration des nicht umgesetzten Dibutylamins mit wässriger Salzsäure.

Bevorzugt hat das Isocyanatgruppen-haltige Polymer einen Gehalt an monomeren Diisocyanaten von weniger als 0.5 Gewichts-%, bevorzugt weniger als 0.2 Gewichts-%, insbesondere weniger als 0.1 Gewichts-%, bezogen auf das Isocyanatgruppen-haltige Polymer. Ein solches Polymer ist typischerweise besonders niedrigviskos.

Die härtbare Zusammensetzung umfasst weiterhin mindestens eine Verbindung mit zwei oder mehr 1,3-Ketoestergruppen der Formel (II), 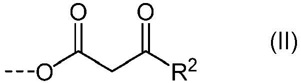 wobei R² für einen einwertigen Kohlenwasserstoffrest mit 1 bis 6 C-Atomen steht.

Bevorzugt ist die Verbindung mit zwei oder mehr 1,3-Ketoestergruppen der Formel (II) bei Raumtemperatur flüssig. Insbesondere hat sie eine Viskosität bei 20 °C von 0.1 bis 50 Pa s, mehr bevorzugt 0.1 bis 30 Pa s, insbesondere 0.1 bis 20 Pa s, gemessen mittels Kegel-Platte Viskosimeter mit Kegeldurchmesser 10 mm, Kegelwinkel 1°, Kegelspitze-Platte-Abstand 0.05 mm, Scherrate 10 s⁻¹. Eine solche Verbindung ermöglicht Zusammensetzungen, die bei Umgebungstemperatur ohne Zusatz von Lösemitteln oder Verdünnern gut verarbeitbar sind.

Bevorzugt weist die Verbindung mit zwei oder mehr 1,3-Ketoestergruppen der Formel (II) zwei bis sechs, besonders bevorzugt zwei bis vier, insbesondere zwei bis drei, 1,3-Ketoestergruppen der Formel (II) auf.

Im Fall einer oligomeren oder polymeren Mischung von Verbindungen mit zwei oder mehr 1,3-Ketoestergruppen der Formel (II) liegt die mittlere Funktionalität in Bezug auf die 1,3-Ketoestergruppen der Formel (II) bevorzugt im Bereich von 1.5 bis 4, besonders bevorzugt 1.8 bis 3, insbesondere 2 bis 3.

Bevorzugt hat die Verbindung mit zwei oder mehr 1,3-Ketoestergruppen der Formel (II) ein Molekulargewicht oder, im Fall einer oligomeren oder polymeren Mischung von Verbindungen mit zwei oder mehr 1,3-Ketoestergruppen der Formel (II), ein mittleres Molekulargewicht Mₙ von 230 bis 10'000 g/mol, besonders bevorzugt 250 bis 6'000 g/mol, insbesondere 500 bis 3'000 g/mol.

Bevorzugt steht R² in Formel (II) für einen Alkylrest mit 1 bis 6 C-Atomen oder für Phenyl, besonders bevorzugt für Methyl, Ethyl, Propyl, Isopropyl, Butyl oder Phenyl.

Am meisten bevorzugt steht R² in Formel (II) für Methyl und die Ketoestergruppen der Formel (II) sind somit Acetoacetatgruppen. Verbindungen mit Acetoacetatgruppen sind besonders einfach zugänglich und ermöglichen Zusammensetzungen mit besonders schneller Aushärtung.

Die Verbindung mit zwei oder mehr 1,3-Ketoestergruppen der Formel (II) wird bevorzugt erhalten aus der Umesterung von mindestens einer Verbindung mit zwei oder mehr Hydroxylgruppen und mindestens einem 1,3-Ketoester der Formel (V), 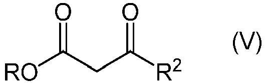 wobei R für C₁₋₆ Alkyl steht und R² die bereits genannten Bedeutungen aufweist. Bevorzugt steht R für Methyl, Ethyl oder tert-Butyl, besonders bevorzugt für Ethyl oder tert-Butyl, insbesondere für Ethyl.

Bevorzugt erfolgt die Umesterung bei einer Temperatur im Bereich von 50 bis 150 °C unter destillativer Entfernung des freigesetzten Alkohols ROH und gegebenenfalls Fragmentierungsprodukten davon, gegebenenfalls unter Vakuum und gegebenenfalls in Anwesenheit eines geeigneten Katalysators.

Ebenfalls möglich ist eine Herstellung, bei welcher die Verbindung mit zwei oder mehr Hydroxylgruppen mit Diketen oder dem Addukt von Diketen mit Aceton (= 2,2,6-Trimethyl-4H-1,3-dioxin-4-on) umgesetzt wird, wobei im Fall des Aceton-Diketen-Addukts Aceton freigesetzt wird.

Als Verbindung mit zwei oder mehr Hydroxylgruppen geeignet sind handelsübliche OH-funktionelle Verbindungen oder Polymere, insbesondere 1,2-Ethandiol, Diethylenglykol, Triethylenglykol, 1,2-Propandiol, Dipropylenglykol, Tripropylenglykol, 1,3-Propandiol, 1,4-Butandiol, 1,2-Butandiol, 2-Methyl-1,3-propandiol, 1,5-Pentandiol, 1,2-Pentandiol, Neopentylglykol, 2-Methyl-1,4-butandiol, 1,6-Hexandiol, 3-Methyl-1,5-pentandiol, 1,8-Octandiol, 2-Ethyl-1,3-hexandiol, 1,10-Decandiol, 1,12-Dodecandiol, Polytetrahydrofurandiole, 1,3-Cyclohexandimethanol, 1,4-Cyclohexandimethanol, Isosorbid, 2,5-Bis(hydroxymethyl)tetrahydrofuran, 2,5(6)-Bis(hydroxymethyl)bicyclo[2.2.1]heptan, 3(4),8(9)-Bis(hydroxymethyl)tricyclo-[5.2.1.0^{2,6}]decan, hydriertes Bisphenol F, hydriertes Bisphenol A, Glycerin, 1,1,1-Trimethylolethan, 1,1,1-Trimethylolpropan, Pentaerythrit, ethoxyliertes oder insbesondere propoxyliertes Glycerin, ethoxyliertes oder insbesondere propoxyliertes 1,1,1-Trimethylolpropan, Poly(oxy-1,2-propylen)diole, EO-terminierte Poly(oxy-1,2-propylen)diole, Poly(oxy-1,2-propylen)triole, EO-terminierte Poly(oxy-1,2-propylen)triole, Di- oder Trimerfettsäure-basierte Polyesterpolyole, Rizinusöl, Derivate von Rizinusöl oder hydroxylierte Pflanzenöle.

Bevorzugt ist die Verbindung mit zwei oder mehr 1,3-Ketoestergruppen der Formel (II) ausgewählt aus der Gruppe bestehend aus 1,2-Propandioldiacetoacetat, Dipropylenglykoldiacetoacetat, Tripropylenglykoldiacetoacetat, 1,3-Propandioldiacetoacetat, 1,4-Butandioldiacetoacetat, 2-Methyl-1,3-propandioldiacetoacetat, 1,5-Pentandioldiacetoacetat, Neopentylglykoldiacetoacetat, 1,6-Hexandioldiacetoacetat, 3-Methyl-1,5-pentandioldiacetoacetat, 2-Ethyl-1,3-hexandioldiacetoacetat, Isosorbid-diacetoacetat, 4,4'-Isopropyliden-bis(cyclohexanol)diacetoacetat, Glycerin-diacetoacetat, Glycerin-triacetoacetat, 1,1,1-Trimethylolethandiacetoacetat, 1,1,1-Trimethylolethantriacetoacetat, 1,1,1-Trimethylolpropandiacetoacetat, 1,1,1-Trimethylolpropantriacetoacetat, dem Triacetoacetat von propoxyliertem 1,1,1-Trimethylolpropan mit insgesamt einem mittleren Molekulargewicht Mₙ von 500 bis 2'000 g/mol, Poly(oxy-1,2-propylen)dioldiacetoacetat mit mittlerem Molekulargewicht Mₙ von 600 bis 5'000 g/mol, Poly(oxy-1,2-propylen)trioltriacetoacetat mit mittlerem Molekulargewicht Mₙ von 2'000 bis 6'000 g/mol, Ethylenoxid-Einheiten enthaltendem Poly(oxy-1,2-propylen)triol-triacetoacetat mit mittlerem Molekulargewicht Mₙ von 2'000 bis 6'000 g/mol, Dimerfettsäure-basiertem Polyesterdioldiacetoacetat mit mittlerem Molekulargewicht Mₙ von 1'000 bis 2'500 g/mol, Trimerfettsäure-basiertem Polyestertrioltriacetoacetat mit mittlerem Molekulargewicht Mₙ von 1'000 bis 3'000 g/mol, Rizinusöl-diacetoacetat und Rizinusöl-triacetoacetat.

Davon bevorzugt ist 2-Methyl-1,3-propandioldiacetoacetat, 1,5-Pentandioldiacetoacetat, Neopentylglykoldiacetoacetat, 1,6-Hexandioldiacetoacetat, 3-Methyl-1,5-pentandioldiacetoacetat, 2-Ethyl-1,3-hexandioldiacetoacetat, 1,1,1-Trimethylolpropandiacetoacetat, 1,1,1-Trimethylolpropantriacetoacetat, ein Triacetoacetat von propoxyliertem 1,1,1-Trimethylolpropan mit insgesamt einem mittleren Molekulargewicht Mₙ von 500 bis 2'000 g/mol, bevorzugt 500 bis 600 g/mol, Dimerfettsäure-basiertes Polyesterdioldiacetoacetat mit mittlerem Molekulargewicht Mₙ von 1'000 bis 2'500 g/mol oder Trimerfettsäure-basiertes Polyestertrioltriacetoacetat mit mittlerem Molekulargewicht Mₙ von 1'000 bis 3'000 g/mol.

Besonders bevorzugt ist ein Triacetoacetat von propoxyliertem 1,1,1-Trimethylolpropan mit insgesamt einem mittleren Molekulargewicht Mₙ von 500 bis 600 g/mol, 2-Methyl-1,3-propandioldiacetoacetat, Neopentylglykoldiacetoacetat, 1,6-Hexandioldiacetoacetat, 3-Methyl-1,5-pentandioldiacetoacetat, 2-Ethyl-1,3-hexandioldiacetoacetat oder ein Dimerfettsäure-basiertes Polyesterdioldiacetoacetat mit mittlerem Molekulargewicht Mₙ von 1'000 bis 2'500 g/mol.

Ganz besonders bevorzugt ist ein Triacetoacetat von propoxyliertem 1,1,1-Trimethylolpropan mit insgesamt einem mittleren Molekulargewicht Mₙ von 500 bis 600 g/mol.

Bevorzugt liegt das Verhältnis der Anzahl 1,3-Ketoestergruppen der Formel (II) zur Anzahl Endgruppen der Formel (I) in der härtbaren Zusammensetzung bei 1 bis 4, besonders bevorzugt bei 1.5 bis 3.5, insbesondere bei 2 bis 3. Eine solche Zusammensetzung härtet schnell und störungsfrei zu einem ausgehärteten Polymer mit hoher Festigkeit und Dehnbarkeit.

Bevorzugt liegt das Verhältnis der Anzahl 1,3-Ketoestergruppen der Formel (II) zur Anzahl Aldimingruppen in der härtbaren Zusammensetzung bei 1 bis 4, besonders bevorzugt bei 1.5 bis 3.5, insbesondere bei 2 bis 3.

Die härtbare Zusammensetzung kann zusätzlich weitere Bestandteile enthalten, insbesondere
- Füllstoffe, insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, Kaoline, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, industriell hergestellte Russe, Graphit, gemahlene Füllstoffe aus landwirtschaftlichen Quellen wie insbesondere Olivenkernmehl oder Nussschalenmehl, Metall-Pulver, beispielsweise von Aluminium, Kupfer, Eisen, Silber oder Stahl, PVC-Pulver oder Hohlkugeln,
- Fasern, insbesondere Glasfasern, Kohlefasern, Metallfasern, Keramikfasern, Hanffasern, Zellulosefasern oder Kunststofffasern wie Polyamidfasern, Polyethylenfasern oder Polypropylenfasern,
- Nanofüllstoffe oder Nanofasen wie Graphen oder Carbon Nanotubes,
- Farbstoffe,
- Pigmente, insbesondere Titandioxid, Chromoxid, Eisenoxide oder organische Pigmente,
- Weichmacher, insbesondere Phthalate, insbesondere Diisononylphthalat (DINP), Diisodecylphthalat (DIDP) oder Di(2-propylheptyl)phthalat (DPHP), hydrierte Phthalate, insbesondere Diisononyl-1,2-cyclohexandicarboxylat (DINCH), Terephthalate, insbesondere Bis(2-ethylhexyl)terephthalat oder Diisononylterephthalat (DINT), hydrierte Terephthalate, insbesondere Bis(2-ethylhexyl)-1,4-cyclohexandicarboxylat oder Diisononyl-1,4-cyclohexandicarboxylat, Isophthalate, Trimellitate, Adipate, insbesondere Dioctyladipat (DOA), Azelate, Sebacate, Citrate, Benzoate, Glycolether, Glycolester, Weichmacher mit Polyetherstruktur, insbesondere Poly(oxy-1,2-propylen)-monole, -diole oder -triole, gegebenenfalls mit blockierten Hydroxylgruppen, insbesondere in der Form von Acetylgruppen, sowie organische Sulfonate oder Phosphate, insbesondere Diphenylkresylphosphat (DPK) oder Tris-2-ethyl-hexylphosphat (TOF), Polybutene, Polyisobutene oder von natürlichen Fetten oder Ölen abgeleitete Weichmacher, insbesondere epoxidiertes Soja- oder Leinöl oder Rapsölmethylester, wobei Phthalate, hydrierte Phthalate, Adipate oder Weichmacher mit Polyetherstruktur bevorzugt sind,
- Lösemittel,
- weitere Aldimine, insbesondere niedrigmolekulare Mono- oder Polyaldimine,
- weitere 1,3-Ketoester, insbesondere monofunktionelle 1,3-Ketoester,
- Katalysatoren für die Reaktion von 1,3-Ketoestergruppen mit Aldimingruppen,
- Modifizierungsmittel wie Kohlenwasserstoffharze, natürliche oder synthetische Wachse oder Bitumen,
- Rheologie-Modifizierer, insbesondere Harnstoffverbindungen, Schichtsilikate wie Bentonite, Derivate von Rizinusöl, hydriertes Rizinusöl, Polyamide, Polyurethane, pyrogene Kieselsäuren oder hydrophob modifizierte Polyoxyethylene,
- Trocknungsmittel, insbesondere Molekularsiebe, Calciumoxid, Mono-Oxazolidine wie Incozol^{®} 2 (von Incorez), Orthoester oder Alkoxysilane,
- Haftvermittler, insbesondere Titanate oder Organoalkoxysilane wie Aminosilane, Mercaptosilane, Epoxysilane, (Meth)acrylsilane, Carbamatosilane, Alkylsilane, S-(Alkylcarbonyl)mercaptosilane oder oligomere Formen dieser Silane,
- nicht-reaktive thermoplastische Polymere, wie Homo- oder Copolymere von ungesättigten Monomeren, insbesondere aus der Gruppe umfassend Ethylen, Propylen, Butylen, Isobutylen, Isopren, Styrol, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO),
- flammhemmende Substanzen, insbesondere die bereits genannten Füllstoffe Aluminiumhydroxid oder Magnesiumhydroxid, organische Phosphorsäureester, Ammoniumpolyphosphate, expandierender Graphit, Melaminverbindungen, Borverbindungen oder Antimonverbindungen,
- Additive, insbesondere Netzmittel, Verlaufmittel, Entschäumer, Entlüfter, Stabilisatoren gegen Oxidation, Wärme, Licht oder UV-Strahlung oder Biozide,
sowie weitere üblicherweise in härtbaren Zusammensetzungen eingesetzte Substanzen.

Bevorzugt enthält die härtbare Zusammensetzung mindestens einen weiteren Bestandteil ausgewählt aus Füllstoffen, Pigmenten, Weichmachern und Haftvermittlern.

Die härtbare Zusammensetzung enthält bezogen auf die gesamte Zusammensetzung bevorzugt weniger als 10 Gewichts-%, besonders bevorzugt weniger als 5 Gewichts-%, insbesondere weniger als 1 Gewichts-%, flüchtige organische Lösemittel mit einem Siedepunkt bei Normaldruck von weniger als 250 °C. Eine solche Zusammensetzung verursacht besonders wenig Emissionen.

Die härtbare Zusammensetzung ist bevorzugt nicht wasserbasiert, das heisst sie liegt weder als Lösung noch als Emulsion oder Dispersion in Wasser vor. Sie ist bevorzugt weitgehend frei von Wasser oder enthält nur einen geringen Gehalt an Wasser. Eine solche Zusammensetzung härtet unabhängig von der Umgebungsfeuchte schnell aus, kann in dicken Schichten und/oder zwischen wasserdichten Substraten eingesetzt werden und zeigt kaum Schwund bei der Aushärtung. Bevorzugt enthält die härtbare Zusammensetzung weniger als 10 Gewichts-%, bevorzugt weniger als 5 Gewichts-%, insbesondere weniger als 2 Gewichts-%, Wasser bezogen auf die gesamte Zusammensetzung.

Insbesondere enthält die härtbare Zusammensetzung weniger als 10 Gewichts-%, bevorzugt weniger als 5 Gewichts-%, insbesondere weniger als 1 Gewichts-%, flüchtige organische Lösemittel mit einem Siedepunkt bei Normaldruck von weniger als 250 °C und weniger als 10 Gewichts-%, bevorzugt weniger als 5 Gewichts-%, insbesondere weniger als 2 Gewichts-%, Wasser bezogen auf die gesamte Zusammensetzung.

Die härtbare Zusammensetzung ist bevorzugt frei von Isocyanatgruppen.

In einer bevorzugten Ausführungsform der Erfindung enthält die härtbare Zusammensetzung mindestens einen Weichmacher, mindestens einen Füllstoff, zusätzlich entweder Russ oder ein Titandioxid, und gegebenenfalls mindestens einen Haftvermittler. Eine solche Zusammensetzung ist besonders geeignet als elastischer Klebstoff oder Dichtstoff mit guter Beständigkeit gegenüber Witterungseinflüssen und UV-Licht. Für den Fall, dass ein Titandioxid enthalten ist, kann zusätzlich eine kleine Menge Russ vorhanden sein, so dass die ausgehärtete Zusammensetzung einen grauen Farbton aufweist.

Bevorzugt wird die härtbare Zusammensetzung als zweikomponentige Zusammensetzung umfassend zwei voneinander getrennt verpackte Komponenten eingesetzt. Dabei sind die Polymere mit Endgruppen der Formel (I) Bestandteil der ersten Komponente und die Verbindungen mit zwei oder mehr 1,3-Ketoestergruppen der Formel (II) Bestandteil der zweiten Komponente.

Weitere Inhaltsstoffe der Zusammensetzung können als Bestandteil der ersten und/oder der zweiten Komponente vorhanden sein, wobei mit Endgruppen der Formel (I) reaktive Bestandteile bevorzugt als Bestandteil der zweiten Komponente vorliegen, und mit 1,3-Ketoestergruppen der Formel (II) reaktive Bestandteile bevorzugt als Bestandteil der ersten Komponente vorliegen.

Jede der beiden Komponenten ist in einer geeigneten Verpackung für sich allein lagerstabil. Zur Verwendung werden die Komponenten kurz vor oder während der Applikation miteinander vermischt, womit die Aushärtung beginnt.

Die erste und die zweite Komponente der härtbaren Zusammensetzung werden getrennt voneinander hergestellt. Dabei werden die Inhaltsstoffe der jeweiligen Komponente miteinander vermischt, so dass eine makroskopisch homogene Masse entsteht.

Die erste und die zweite Komponente werden in Bezug auf ihre Konsistenz bevorzugt so eingestellt, dass sie bei Umgebungsbedingungen mit einfachen Verfahren gut, d.h. möglichst schnell und vollständig, miteinander vermischt werden können. Bevorzugt sind die beiden Komponenten flüssig oder pastös und haben eine ähnliche Viskosität.

Die erste und die zweite Komponente werden jeweils in einem separaten Gebinde gelagert. Geeignete Gebinde sind insbesondere Fässer, Container, Hobbocks, Eimer, Kanister, Dosen, Beutel, Schlauchbeutel, Kartuschen oder Tuben.

Zur Verwendung der härtbaren Zusammensetzung werden die Komponenten kurz vor oder während der Applikation miteinander vermischt. Das Mischungsverhältnis wird dabei bevorzugt so gewählt, dass das Verhältnis der Anzahl 1,3-Ketoestergruppen der Formel (II) zur Anzahl Endgruppen der Formel (I) im bevorzugten Bereich liegt. In Gewichtsteilen liegt das Mischungsverhältnis zwischen der ersten und der zweiten Komponente typischerweise bei etwa 100:1 bis 1:1, insbesondere 50:1 bis 1:1.

Werden die Komponenten vor der Applikation miteinander vermischt, so muss darauf geachtet werden, dass zwischen dem Vermischen der Komponenten und der Applikation nicht zu viel Zeit vergeht, da es sonst durch die einsetzende Reaktion und dem damit verbundenen Anstieg der Viskosität zu Störungen wie beispielsweise einem ungenügenden Applizierbarkeit oder einer verlangsamten oder unvollständigen Haftung zum Substrat kommen kann. Insbesondere soll die Offenzeit der Zusammensetzung während der Applikation nicht überschritten werden.

Als "Offenzeit" wird die Zeitspanne zwischen dem Vermischen der Komponenten und dem Ende eines für die Verarbeitung geeigneten Zustands der Zusammensetzung bezeichnet.

Das Vermischen erfolgt bevorzugt bei Umgebungstemperatur, bevorzugt bei einer Temperatur im Bereich von -5 bis 50 °C, bevorzugt 0 bis 40 °C, insbesondere 5 bis 35 °C.

Mit dem Vermischen der Inhaltsstoffe bzw. der Komponenten beginnt die Zusammensetzung durch die einsetzende chemische Reaktion auszuhärten. Dabei reagieren insbesondere vorhandene 1,3-Ketoestergruppen der Formel (II) mit den Endgruppen der Formel (I). Es kann vermutet werden, dass typischerweise zwei Ketoestergruppen mit einer Endgruppe der Formel (I) unter Freisetzung von Wasser zu einer cyclischen Struktureinheit reagieren. Dabei wird kein Aldehyd freigesetzt.

Die Aushärtung erfolgt bevorzugt bei Umgebungstemperatur, bevorzugt bei einer Temperatur im Bereich von -5 bis 50°C, insbesondere 0 bis 40°C.

Ein weiterer Gegenstand der Erfindung ist die ausgehärtete Zusammensetzung, erhalten aus der härtbaren Zusammensetzung nach dem Vermischen der Inhaltsstoffe bzw. der Komponenten.

Bevorzugt weist die ausgehärtete Zusammensetzung eine Zugfestigkeit von mindestens 4 MPa auf, bestimmt gemäss DIN EN 53504 bei einer Zuggeschwindigkeit von 200 mm/min an hantelförmigen Prüfkörpern mit einer Dicke von 2 mm und einer Länge von 75 mm bei einer Steglänge von 30 mm und einer Stegbreite von 4 mm.

Bevorzugt weist die ausgehärtete Zusammensetzung eine Bruchdehnung von mindestens 200 %, insbesondere mindestens 300 %, auf, bestimmt gemäss DIN EN 53504 bei einer Zuggeschwindigkeit von 200 mm/min an hantelförmigen Prüfkörpern mit einer Dicke von 2 mm und einer Länge von 75 mm bei einer Steglänge von 30 mm und einer Stegbreite von 4 mm.

Bevorzugt weist die ausgehärtete Zusammensetzung einen Weiterreisswiderstand von mindestens 4.5 N/mm, insbesondere mindestens 5 N/mm, auf, bestimmt gemäss DIN ISO 34-1 Verfahren B (winkelförmiger Prüfkörper) bei einer Probendicke von 2 mm und einer Zuggeschwindigkeit von 500 mm/min.

Bevorzugt beträgt die Zugfestigkeit der ausgehärteten Zusammensetzung mindestens 4 MPa und/oder die Bruchdehnung beträgt mindestens 200 %, bevorzugt mindestens 300 %, bestimmt gemäss DIN EN 53504 bei einer Zuggeschwindigkeit von 200 mm/min an hantelförmigen Prüfkörpern mit einer Dicke von 2 mm und einer Länge von 75 mm bei einer Steglänge von 30 mm und einer Stegbreite von 4 mm.

Die härtbare Zusammensetzung eignet sich für eine Vielzahl von Verwendungen. Sie ist insbesondere geeignet für Verwendungen, bei welchen elastische Eigenschaften mit einer hohen Dehnbarkeit gefordert sind.

Ein weiterer Gegenstand der Erfindung ist die Verwendung der härtbare Zusammensetzung als elastischer Klebstoff, elastischer Dichtstoff oder elastische Beschichtung, wobei die Inhaltsstoffe bzw. die Komponenten der Zusammensetzung miteinander vermischt werden und die vermischte Zusammensetzung im flüssigen Zustand auf mindestens ein Substrat appliziert wird.

Aufgrund der hohen Dehnbarkeit besonders bevorzugt ist die Verwendung als elastischer Klebstoff oder elastischer Dichtstoff.

Geeignete Substrate sind insbesondere:
- Glas, Glaskeramik, Beton, Mörtel, Zementestrich, Faserzement, Backstein, Ziegel, Gips oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Eisen, Stahl, Kupfer, weitere Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- faserverstärkte Kunststoffe, wie Kohlefaser-verstärkte Kunststoffe (CFK), Glasfaser-verstärkte Kunststoffe (GFK), Naturfaser-verstärkte Kunststoffe (NFK) und Sheet Moulding Compounds (SMC);
- Isoliermaterialen, insbesondere Schäume, insbesondere aus EPS, XPS, PUR, PIR, Aerogel oder geschäumtes Glas (Foamglas), oder Fasern aus Steinwolle oder Glaswolle,
- beschichtete oder lackierte Substrate, insbesondere lackierte Fliesen, gestrichener Beton, pulverbeschichtete Metalle oder Legierungen oder lackierte Bleche;
- Beschichtungen, Farben oder Lacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Aus der Verwendung der härtbaren Zusammensetzung wird ein Artikel erhalten. Der Artikel ist insbesondere mit der härtbaren Zusammensetzung verklebt, abgedichtet oder beschichtet. Dieser Artikel kann ein Bauwerk oder ein Teil davon sein, insbesondere ein Bauwerk des Hoch- oder Tiefbaus, eine Brücke, ein Dach, ein Treppenhaus oder eine Fassade, oder er kann ein industrielles Gut oder ein Konsumgut sein, insbesondere ein Fenster, ein Rohr, ein Rotorblatt einer Windkraftanlage, eine Haushaltmaschine oder ein Verkehrsmittel wie insbesondere ein Automobil, ein Bus, ein Lastkraftwagen, ein Schienenfahrzeug, ein Schiff, ein Flugzeug oder ein Helikopter oder ein Anbauteil davon.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" ("NK") wird eine Temperatur von 23±1°C und eine relative Luftfeuchtigkeit von 50±5% bezeichnet.

Die verwendeten Chemikalien waren, sofern nicht anders bezeichnet, von Merck.

Die **Viskosität** wurde auf einem thermostatisierten Kegel-Platte-Viskosimeter Rheotec RC30 (Kegeldurchmesser 10 mm, Kegelwinkel 1°, Kegelspitze-Platte-Abstand 0.05 mm, Scherrate 10 s⁻¹) gemessen.

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen.

Der **Gehalt an monomerem Diisocyanat** wurde bei allen Isocyanatgruppen-haltigen Polymeren mittels HPLC (Detektion über Photodiodenarray; 0.04 M Natriumacetat / Acetonitril als mobile Phase) nach vorgängiger Derivation mittels N-Propyl-4-nitrobenzylamin bestimmt.

### Herstellung von OH-funktionellen Aldiminen:

### Aldimin-1: N-Benzyliden-2-(2-aminoethoxy)ethanol

105.0 g (1 mol) 2-(2-Aminoethoxy)ethanol (Diglycolamine^{®} Agent, von Huntsman) wurde unter Stickstoffatmosphäre vorgelegt, unter gutem Rühren mit 106.1 g (1 mol) Benzaldehyd versetzt und anschliessend die flüchtigen Bestandteile bei 80 °C und 10 mbar Vakuum entfernt. Erhalten wurden 192.0 g N-Benzyliden-2-(2-aminoethoxy)ethanol als klare, gelbliche Flüssigkeit.

### Aldimin-2: N-(5-Methylfurfuryliden)-2-(2-aminoethoxy)ethanol

52.5 g (0.5 mol) 2-(2-Aminoethoxy)ethanol (Diglycolamine^{®} Agent, von Huntsman) wurde unter Stickstoffatmosphäre vorgelegt, unter gutem Rühren mit 55.1 g (0.5 mol) 5-Methylfurfural versetzt und anschliessend die flüchtigen Bestandteile bei 80 °C und 10 mbar Vakuum entfernt. Erhalten wurden 98.1 g N-(5-Methylfurfuryliden)-2-(2-aminoethoxy)ethanol als klare, gelbliche Flüssigkeit.

### Aldimin-3: Mischung aus N-(2-(2-hydroxyethoxy)benzyliden)butanamin und N-(2-(2-(2-hydroxyethoxy)ethoxy)benzyliden)butanamin

72.7 g (0.4 mol Aldehydgruppen) einer Mischung aus 2-(2-Hydroxyethoxy)benzaldehyd und 2-(2-(2-Hydroxyethoxy)ethoxy)benzaldehyd wurde unter Stickstoffatmosphäre vorgelegt und auf 50 °C erwärmt. Dann wurden 29.9 g (0.4 mol) Butylamin unter gutem Rühren langsam zugegeben, anschliessend während 30 min bei 60 °C gerührt und dann die flüchtigen Bestandteile bei 80 °C und 10 mbar Vakuum entfernt. Erhalten wurden 95.2 g einer Mischung aus N-(2-(2-hydroxyethoxy)benzyliden)butan-1-amin und N-(2-(2-(2-hydroxyethoxy)ethoxy)benzyliden)butan-1-amin als klare, orange Flüssigkeit mit einem berechneten mittleren Aldimin-Equivalentgewicht von 243.3 g/eq.

### Herstellung von Isocyanatgruppen-haltigen Polymeren:

### Polymer NCO-1:

780 g Ethylenoxid-terminiertes Polyoxypropylentriol (Desmophen^{®} 5031 BT, OH-Zahl 28.0 mg KOH/g, OH-Funktionalität ca. 2.3, von Covestro) und 303 g Isophorondiisocyanat (Vestanat^{®} IPDI, von Evonik) wurden bei 80 °C nach bekanntem Verfahren zu einer Reaktionsmischung mit einem NCO-Gehalt von 9.1 Gewichts-% umgesetzt. Anschliessend wurden die flüchtigen Bestandteile, insbesondere nicht umgesetztes Isophorondiisocyanat, in einem Kurzwegverdampfer destillativ entfernt (Manteltemperatur 160 °C, Druck 0.1 bis 0.005 mbar), wobei ein Polymer mit einem NCO-Gehalt von 1.8 Gewichts-% und einem Gehalt an monomerem Isophorondiisocyanat von 0.02 Gewichts-% erhalten wurde.

### Polymer NCO-2:

818 g Polyoxypropylendiol (Acclaim^{®} 4200, OH-Zahl 28.5 mg KOH/g, von Covestro) und 227 g Isophorondiisocyanat (Vestanat^{®} IPDI, von Evonik) wurden bei 80 °C nach bekanntem Verfahren zu einer Reaktionsmischung mit einem NCO-Gehalt von 6.6 Gewichts-% umgesetzt. Anschliessend wurden die flüchtigen Bestandteile, insbesondere nicht umgesetztes Isophorondiisocyanat, in einem Kurzwegverdampfer destillativ entfernt (Manteltemperatur 160 °C, Druck 0.1 bis 0.005 mbar), wobei ein Polymer mit einem NCO-Gehalt von 1.9 Gewichts-% und einem Gehalt an monomerem Isophorondiisocyanat von 0.03 Gewichts-% erhalten wurde.

### Herstellung eines Aldehyd-funktionellen Polymers:

### Polymer CHO-1:

500 g Polymer **NCO-1** (214 mmol NCO-Gruppen) wurde in Gegenwart von 0.1 g Dibutylzinndilaurat unter Ausschluss von Feuchtigkeit bei 80 °C mit 27.7 g (220 mmol) 5-Hydroxymethylfurfural umgesetzt, bis mittels IR-Spektroskopie keine Isocyanatgruppen mehr nachweisbar waren. Das erhaltene Aldehyd-funktionelle Polymer hatte eine Viskosität von 64 Pa s bei 20 °C und ein berechnetes Aldehyd-Equivalentgewicht von 2400 g/eq.

### Herstellung von Aldimin-funktionellen Polymeren:

### Polymer A1:

400 g Polymer **NCO-1** (171 mmol NCO-Gruppen) wurde in Gegenwart von 0.1 g Dibutylzinndilaurat unter Ausschluss von Feuchtigkeit bei 80 °C mit 34.6 g (179 mmol) **Aldimin-1** umgesetzt, bis mittels IR-Spektroskopie keine Isocyanatgruppen mehr nachweisbar waren. Das erhaltene Aldimin-funktionelle Polymer hatte eine Viskosität von 143 Pa s bei 20 °C und ein berechnetes Aldimin-Equivalentgewicht von 2428 g/eq.

### Polymer A2:

400 g Polymer **NCO-1** (171 mmol NCO-Gruppen) wurde in Gegenwart von 0.1 g Dibutylzinndilaurat unter Ausschluss von Feuchtigkeit bei 80 °C mit 35.3 g (179 mmol) **Aldimin-2** umgesetzt, bis mittels IR-Spektroskopie keine Isocyanatgruppen mehr nachweisbar waren. Das erhaltene Aldimin-funktionelle Polymer hatte eine Viskosität von 194 Pa s bei 20 °C und ein berechnetes Aldimin-Equivalentgewicht von 2432 g/eq.

### Polymer A3:

400 g Polymer **CHO-1** (167 mmol Aldehydgruppen) wurde mit 12.2 g (167 mmol) Butylamin versetzt, während 30 min bei 50 °C gerührt und anschliessend bei 90 °C im Vakkum das freigesetzte Wasser entfernt. Das erhaltene Aldimin-funktionelle Polymer hatte eine Viskosität von 120 Pa s bei 20 °C und ein berechnetes Aldimin-Equivalentgewicht von 2416 g/eq.

### Polymer A4:

400 g Polymer **NCO-1** (171 mmol NCO-Gruppen) wurde in Gegenwart von 0.1 g Dibutylzinndilaurat unter Ausschluss von Feuchtigkeit bei 80 °C mit 43.5 g (179 mmol) **Aldimin-3** umgesetzt, bis mittels IR-Spektroskopie keine Isocyanatgruppen mehr nachweisbar waren. Das erhaltene Aldimin-funktionelle Polymer hatte eine Viskosität von 127 Pa s bei 20 °C und ein berechnetes Aldimin-Equivalentgewicht von 2478 g/eq.

### Polymer A5:

400 g Polymer **NCO-2** (180 mmol NCO-Gruppen) wurde in Gegenwart von 0.1 g Dibutylzinndilaurat unter Ausschluss von Feuchtigkeit bei 80 °C mit 35.5 g (184 mmol) **Aldimin-1** umgesetzt, bis mittels IR-Spektroskopie keine Isocyanatgruppen mehr nachweisbar waren. Das erhaltene Aldimin-funktionelle Polymer hatte eine Viskosität von 111 Pa s bei 20 °C und ein berechnetes Aldimin-Equivalentgewicht von 2367 g/eq.

### Polymer R1:

400 g (0.21 mol NH₂) Polyoxypropylentriamin (Jeffamine^{®} T-5000, von Huntsman) wurde unter Stickstoffatmosphäre vorgelegt, unter gutem Rühren mit 22.3 g (0.21 mol) Benzaldehyd versetzt und anschliessend die flüchtigen Bestandteile bei 80 °C und 10 mbar Vakuum entfernt. Erhalten wurde eine klare, gelbliche Flüssigkeit mit einem berechneten Aldimin-Equivalentgewicht von 1993 g/eq.

### Polymer R2:

400 g (0.20 mol NH₂) Polyoxypropylendiamin (Jeffamine^{®} D-4000, von Huntsman) wurde unter Stickstoffatmosphäre vorgelegt, unter gutem Rühren mit 21.2 g (0.20 mol) Benzaldehyd versetzt und anschliessend die flüchtigen Bestandteile bei 80 °C und 10 mbar Vakuum entfernt. Erhalten wurde eine klare, gelbliche Flüssigkeit mit einem berechneten Aldimin-Equivalentgewicht von 2088 g/eq.

Die **Polymere A1** bis **A5** enthalten Endgruppen der Formel (I) mit X = O. Die **Polymere R1** und **R2** enthalten Endgruppen, welche nicht der Formel (I) entsprechen, und dienen als Vergleich.

### Herstellung von Verbindungen mit Ketoestergruppen:

### Verbindung B1:

50 g (0.49 mol OH) eines Polyoxypropylentriols (Desmophen^{®} 4011 T, OH-Zahl 550 mg KOH/g, propoxyliertes 1,1,1-Trimethylolpropan, von Covestro) wurde mit 67 g (0.52 mol) Ethylacetoacetat und 0.1 g Tetra-n-butyltitanat (Tyzor^{®} TnBT, von Dorf Ketal) versetzt und unter Vakuum und Entfernung der flüchtigen Bestandteile bei einer Temperatur von 80 bis 140 °C umgesetzt. Erhalten wurde eine klare, farblose Flüssigkeit mit einer Viskosität bei 20 °C von 0.8 Pa s und einem berechneten Acetoacetat-Equivalentgewicht von 186 g/eq.

### Verbindung B2:

104.1 g (2 mol OH) Neopentylglykol (= 2,2-Dimethyl-1,3-propandiol) wurde mit 286.3 g (2.2 mol) Ethylacetoacetat und 0.4 g Tetra-n-butyltitanat versetzt und unter Vakuum und Entfernung der flüchtigen Bestandteile bei einer Temperatur von 80 bis 140 °C umgesetzt. Erhalten wurde eine klare, gelbliche Flüssigkeit mit einer Viskosität bei 20 °C von < 0.05 Pa·s und einem berechneten Acetoacetat-Equivalentgewicht von 136 g/eq.

### Verbindung B3:

500 g (1.1 mol OH) amorphes Dimerfettsäure-basiertes Polyesterdiol mit OH-Zahl 123 mg KOH/g wurde mit 156.2 g (1.2 mol) Ethylacetoacetat und 0.6 g Tetra-n-butyltitanat (Tyzor^{®} TnBT, von Dorf Ketal) versetzt und unter Vakuum und Entfernung der flüchtigen Bestandteile bei einer Temperatur von 80 bis 140 °C umgesetzt. Erhalten wurden 608.7 g einer klaren, gelblichen Flüssigkeit mit einer Viskosität bei 20 °C von 14.4 Pa·s und einem berechneten Acetoacetat-Equivalentgewicht von 540 g/eq.

### Herstellung von härtbaren Zusammensetzungen,

### Zusammensetzungen Z1 bis Z18:

Für jede Zusammensetzung wurden die in den Tabellen 1 bis 3 angegebenen Inhaltsstoffe der Komponente **K1** in den angegebenen Mengen (in Gewichtsteilen) mittels eines Zentrifugalmischers (Thinky ARE-250, 2000 U/min, 60 s) vermischt und in einem verschlossenen Gebinde aufbewahrt.

Als "CaCOs gefällt" wurde Socal^{®} U1S2 (von Imerys), ein gefälltes und mit Stearat beschichtetes Calciumcarbonat, eingesetzt.

Als "Russ" wurde Monarch^{®} 570 (von Cabot) eingesetzt.

Weiterhin wurden die in den Tabellen 1 bis 3 angegebenen Inhaltsstoffe der Komponente **K2** in den angegebenen Mengen (in Gewichtsteilen) vermischt und in einem verschlossenen Gebinde aufbewahrt.

Anschliessend wurden die Komponenten **K1** und **K2** jeder Zusammensetzung mittels des Zentrifugalmischers vermischt und wie folgt beschrieben geprüft.

Die Zeit bis zur Klebefreiheit **TFT** (Tack Free Time) wurde bestimmt, indem 20 g der vermischten Zusammensetzung in einer Schichtdicke von ca. 2 mm auf Pappkarton aufgetragen und im Normklima die Zeitdauer bestimmt wurde, bis beim leichten Antippen der Oberfläche der applizierten Zusammensetzung mittels einer Pipette aus LDPE erstmals keine Rückstände auf der Pipette mehr zurückblieben.

Zur Bestimmung der mechanischen Eigenschaften wurde die vermischte Zusammensetzung auf einem silikonbeschichteten Trennpapier zu einem Film von 2 mm Dicke aufgebracht, dieser während 7 Tagen im Normklima aushärten lassen, einige hantelförmige Prüfkörper mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit, Bruchdehnung, E-Modul 5%** (bei 0.5-5% Dehnung) und **E-Modul 50%** (bei 0.5-50% Dehnung) geprüft. Weiter wurden einige Prüfkörper zur Bestimmung des **Weiterreisswiderstands** ausgestanzt und gemäss DIN ISO 34-1, Verfahren B (winkelförmiger Prüfkörper) bei einer Zuggeschwindigkeit von 500 mm/min geprüft. Die **Shore A** Härte wurde nach DIN 53505 an während 7 Tagen im Normklima gehärteten Prüfkörpern (Durchmesser 20 mm, Dicke 5 mm) bestimmt. Diese Resultate sind mit dem Zusatz **"7d NK"** versehen. Zur Bestimmung der Beständigkeit gegen Hitze und Wasser wurden weitere Shore A-Prüfkörper nach 7 Tagen Aushärtung im Normklima entweder zusätzlich während 7 Tagen in einem Umluftofen bei 100 °C gelagert, oder zusätzlich während 7 Tagen bei 70 °C und 100 % relativer Feuchtigkeit gelagert, und davon jeweils nach Abkühlen auf Raumtemperatur die Shore A-Härte bestimmt. Diese Resultate sind mit dem Zusatz **"+7d 100°C"** bzw. **"+7d 70/100"** versehen.

Alle Zusammensetzungen härteten vollständig geruchsfrei aus. Dabei entstand jeweils ein elastisches Polymer mit ebenmässiger, klebfreier Oberfläche.

Die Resultate sind in den Tabellen 1 bis 3 angegeben.

Die mit **"(Ref.)"** bezeichneten Zusammensetzungen sind Vergleichsbeispiele.

**Tabelle 1: Zusammensetzung und Eigenschaften von Z1 bis Z8. ¹ 1,8-Diazabicyclo[5.4.0]undec-7-en (Lupragen^{®} N700, von BASF) ² Verhältnis der Anzahl Acetoacetatgruppen zur Anzahl Aldimingruppen**

| **Zusammensetzung** | | **Z1** | **Z2** | **Z3** | **Z4** | **Z5** | **Z6** | **Z7 (Ref.)** | **Z8 (Ref.)** |
|---|---|---|---|---|---|---|---|---|---|
| **Komponente K1:** | | | | | | | | | |
| | **Polymer A1** | 30.0 | 30.0 | 30.0 | 30.0 | 30.0 | 30.0 | - | - |
| | **Polymer CHO-1** | - | - | - | - | - | - | 30.0 | 30.0 |
| | Diisodecylphthalat | 20.0 | 20.0 | 20.0 | 20.0 | 20.0 | 20.0 | 20.0 | 20.0 |
| | CaCO₃ gefällt | 30.0 | 30.0 | 30.0 | 30.0 | 30.0 | 30.0 | 30.0 | 30.0 |
| | Russ | 10.0 | 10.0 | 10.0 | 10.0 | 10.0 | 10.0 | 10.0 | 10.0 |
| | DBU¹ | - | - | - | - | - | - | 0.3 | 0.3 |

| **Komponente K2:** | | | | | | | | | |
|---|---|---|---|---|---|---|---|---|---|
| | Verbindung **B1** | 2.28 | 3.4 | 4.6 | 5.7 | 6.85 | 8.0 | 3.6 | 5.3 |
| | Verhältnis Acac / Aldimingruppen ² | 1.0 | 1.5 | 2.0 | 2.5 | 3.0 | 3.5 | - | - |
| | TFT [h:min] | 3:45 | 2:30 | 2:00 | 1:45 | 1:30 | 1:25 | 0:15 | 1:30 |
| | Zugfestigkeit [MPa] | 3.1 | 5.1 | 5.2 | 5.5 | 5.2 | 4.3 | 3.3 | 4.2 |
| | Bruchdehnung [%] | 360 | 270 | 220 | 225 | 250 | 225 | 157 | 150 |
| | E-Modul 5% [MPa] | 0.97 | 2.7 | 3.8 | 4.2 | 2.8 | 2.5 | 3.9 | 3.7 |
| | E-Modul 50% [MPa] | 0.95 | 2.2 | 3.1 | 3.0 | 2.7 | 2.4 | 2.2 | 3.3 |
| | Weiterreisswiderstand [N/mm] | 7.9 | 5.7 | 5.1 | 5.3 | 6.6 | 5.9 | 3.8 | 4.0 |
| | Shore A (7d NK) | 33 | 51 | 57 | 57 | 55 | 50 | 54 | 60 |
| | (+ 7d 100°C) | 55 | 59 | 64 | 67 | 66 | 62 | 59 | 68 |
| | (+ 7d 70/100) | 39 | 48 | 51 | 50 | 46 | 43 | 53 | 51 |

Aus der Tabelle 1 ist ersichtlich, dass die erfindungsgemässen Zusammensetzungen **Z1** bis **Z6** im Stöchiometriebereich von 1 bis 3.5 Acetoacetatgruppen pro Aldimingruppe zu einem elastischen Polymer aushärteten. Die Vergleichs-Zusammensetzungen **Z7** und **Z8** mit einem Aldehyd-funktionellen Polymer härteten ebenfalls zu einem elastischen Polymer aus, wobei die Dehnbarkeit und der Weiterreisswiderstand aber deutlich geringer waren.

**Tabelle 2: Zusammensetzung und Eigenschaften von Z4 und Z9 bis Z14. ¹ Verhältnis der Anzahl Acetoacetatgruppen zur Anzahl Aldimingruppen**

| **Zusammensetzun g** | | **Z4** | **Z9** | **Z10** | **Z11** | **Z12 (Ref.)** | **Z13** | **Z14 (Ref.)** |
|---|---|---|---|---|---|---|---|---|
| **Komponente K1:** | | | | | | | | |
| | **Polymer** | **A1** | **A2** | **A3** | **A4** | **R1** | **A5** | **R2** |
| | | 30.0 | 30.0 | 30.0 | 30.0 | 26.2 | 30.0 | 27.4 |
| | Diisodecylphthalat | 20.0 | 20.0 | 20.0 | 20.0 | 20.0 | 20.0 | 20.0 |
| | CaCO₃ gefällt | 30.0 | 30.0 | 30.0 | 30.0 | 30.0 | 30.0 | 30.0 |
| | Russ | 10.0 | 10.0 | 10.0 | 10.0 | 10.0 | 10.0 | 10.0 |

| **Komponente K2:** | | | | | | | | |
|---|---|---|---|---|---|---|---|---|
| | Verbindung **B1** | 5.7 | 5.7 | 5.7 | 5.7 | 6.1 | 5.7 | 5.9 |
| Verhältnis Acac / Aldimingruppen ¹ | | 2.5 | 2.5 | 2.5 | 2.5 | 2.5 | 2.4 | 2.4 |
| TFT | | 95 min | 20 min | 10 min | 45 min | 18 h | 18 h | > 24 h |
| Zugfestigkeit [MPa] | | 5.5 | 5.5 | 4.0 | 4.5 | 3.0 | 4.1 | 2.9 |
| Bruchdehnung [%] | | 225 | 310 | 205 | 255 | 130 | 305 | 290 |
| E-Modul 5% [MPa] | | 4.2 | 2.9 | 3.7 | 2.9 | 2.7 | 1.7 | 1.3 |
| E-Modul 50% [MPa] | | 3.0 | 2.3 | 2.5 | 2.3 | 3.0 | 1.6 | 1.3 |
| Weiterreisswiderstand [N/mm] | | 5.3 | 6.5 | 4.7 | 5.0 | 3.2 | 7.5 | 9.5 |
| Shore A (7d NK) | | 57 | 53 | 58 | 56 | 59 | 45 | 42 |
| | (+ 7d 100°C) | 67 | 63 | 67 | 66 | 69 | 58 | 58 |
| | (+ 7d 70/100) | 50 | 44 | 53 | 50 | 52 | 35 | 36 |

Aus der Tabelle 2 ist ersichtlich, dass die erfindungsgemässen Zusammensetzungen **Z4** und **Z9** bis **Z11** eine kurze Zeit bis zur Klebefreiheit, eine hohe Zugfestigkeit, hohe E-Moduli und eine hohe Dehnbarkeit zeigten, während die Vergleichs-Zusammensetzung **Z12 (Ref.),** bei welcher die Endgruppen nicht der Formel (I) entsprechen, eine sehr viel längere Zeit bis zur Klebefreiheit, eine niedrigere Zugfestigkeit, niedrigere E-Moduli und eine geringere Dehnbarkeit zeigte.

Ebenso zeigte ein Vergleich der erfindungsgemässen Zusammensetzung **Z13** auf Basis des linearen Polymers **A5** mit der Vergleichs-Zusammensetzung **Z14** auf Basis des linearen Polymers **R2,** bei welchem die Endgruppen nicht der Formel (I) entsprechen, eine kürzere Zeit bis zur Klebefreiheit, eine höhere Zugfestigkeit, höhere E-Moduli und eine etwas höhere Dehnbarkeit.

**Tabelle 3: Zusammensetzung und Eigenschaften von Z15 bis Z18. ¹ Verhältnis der Anzahl Acetoacetatgruppen zur Anzahl Aldimingruppen**

| **Zusammensetzung** | | **Z15** | **Z16** | **Z17** | **Z18** |
|---|---|---|---|---|---|
| | **Komponente K1:** | | | | |
| | **Polymer A1** | 30.0 | 30.0 | 30.0 | 30.0 |
| | Diisodecylphthalat | 20.0 | 20.0 | 20.0 | 20.0 |
| | CaCO₃ gefällt | 30.0 | 30.0 | 30.0 | 30.0 |
| | Russ | 10.0 | 10.0 | 10.0 | 10.0 |

| | **Komponente K2:** | | | | |
|---|---|---|---|---|---|
| | Verbindung **B1** | 2.9 | - | 2.3 | - |
| | Verbindung **B2** | 2.1 | 4.2 | - | - |
| | Verbindung **B3** | - | - | 6.8 | 13.6 |
| | Verhältnis Acac / Aldimingruppen ¹ | 2.5 | 2.5 | 2.0 | 2.0 |
| | TFT | 3 h | 24 h | 24 h | 24 h |
| | Zugfestigkeit [MPa] | 6.4 | 6.5 | 4.6 | 5.1 |
| | Bruchdehnung [%] | 360 | 590 | 385 | 745 |
| | E-Modul 5% [MPa] | 2.8 | 1.7 | 3.1 | 1.3 |
| | E-Modul 50% [MPa] | 2.1 | 1.3 | 1.8 | 0.7 |
| | Weiterreisswiderstand [N/mm] | 7.3 | 13.2 | 8.6 | 15.0 |
| | Shore A (7d NK) | 55 | 41 | 50 | 33 |
| | (+ 7d 100°C) | 63 | 58 | 63 | 46 |
| | (+ 7d 70/100) | 43 | 28 | 36 | 20 |

## Patentansprüche

1. Härtbare Zusammensetzung umfassend
- mindestens ein Polymer mit Endgruppen der Formel (I) und einem mittleren Molekulargewicht Mₙ von 1'000 bis 30'000 g/mol, 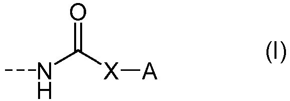 wobei
X für O, S oder NR¹ und R¹ für H oder einen einwertigen Kohlenwasserstoffrest mit 1 bis 18 C-Atomen steht, und
A für einen einwertigen organischen Rest mit 7 bis 30 C-Atomen steht, der eine aromatische Aldimingruppe enthält,
und
- mindestens eine Verbindung mit zwei oder mehr 1,3-Ketoestergruppen der Formel (II), 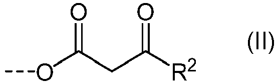 wobei R² für einen einwertigen Kohlenwasserstoffrest mit 1 bis 6 C-Atomen steht.

2. Härtbare Zusammensetzung gemäss Anspruch 1, **dadurch gekennzeichnet, dass** das Verhältnis der Anzahl 1,3-Ketoestergruppen der Formel (II) zur Anzahl Endgruppen der Formel (I) bei 1 bis 4 liegt.

3. Härtbare Zusammensetzung gemäss Anspruch 1 oder 2, **dadurch gekennzeichnet, dass** das Polymer mit Endgruppen der Formel (I) eine mittlere Aldimin-Funktionalität von 1.5 bis 4, bevorzugt 1.8 bis 3, insbesondere 2 bis 3, aufweist.

4. Härtbare Zusammensetzung gemäss einem der Ansprüche 1 bis 3, **dadurch gekennzeichnet, dass** das Polymer mit Endgruppen der Formel (I) ein Polyether-Rückgrat aufweist.

5. Härtbare Zusammensetzung gemäss einem der Ansprüche 1 bis 4, **dadurch gekennzeichnet, dass** X für O steht.

6. Härtbare Zusammensetzung gemäss einem der Ansprüche 1 bis 5, **dadurch gekennzeichnet, dass** A in den Endgruppen der Formel (I) für einen Rest der Formel ---G¹-N=CH-Z oder ---G²-CH=N-R³ steht, wobei,
G¹ für einen zweiwertigen aliphatischen, cycloaliphatischen oder arylaliphatischen, gegebenenfalls Ether-Sauerstoff aufweisenden Kohlenwasserstoff- Rest mit 2 bis 18 C-Atomen steht,
Z für einen aromatischen oder heteroaromatischen fünf- oder sechsgliedrigen Ring steht, der gegebenenfalls substituiert und/oder anelliert ist und insgesamt 4 bis 25 C-Atome umfasst,
G² für einen zweiwertigen, gegebenenfalls Sauerstoffatome enthaltenden arylaliphatischen Kohlenwasserstoffrest mit 5 bis 12 C-Atomen steht, welcher über ein aliphatisches C-Atom an X und über einen aromatischen oder heteroaromatischen Ring an CH=N gebunden ist, und
R³ für einen einwertigen, gegebenenfalls Ether-Sauerstoff enthaltenden aliphatischen, cycloaliphatischen oder arylaliphatischen Kohlenwasserstoffrest mit 1 bis 13 C-Atomen steht.

7. Härtbare Zusammensetzung gemäss einem der Ansprüche 1 bis 6, **dadurch gekennzeichnet, dass** das Polymer mit Endgruppen der Formel (I) erhalten wird durch die Umsetzung eines Isocyanatgruppen-haltigen Polymers mit einem Aldimin der Formel HX-A in einem Verhältnis der Anzahl Mol Isocyanatgruppen zur Anzahl Mol Aldimin der Formel HX-A von mindestens 1.

8. Härtbare Zusammensetzung gemäss einem der Ansprüche 1 bis 7, **dadurch gekennzeichnet, dass** die Verbindung mit zwei oder mehr 1,3-Ketoestergruppen der Formel (II) ein Molekulargewicht oder, im Fall einer oligomeren oder polymeren Mischung von Verbindungen mit zwei oder mehr 1,3-Ketoestergruppen der Formel (II), ein mittleres Molekulargewicht Mₙ von 230 bis 10'000 g/mol, bevorzugt 250 bis 6'000 g/mol, insbesondere 500 bis 3'000 g/mol, hat.

9. Härtbare Zusammensetzung gemäss einem der Ansprüche 1 bis 8, **dadurch gekennzeichnet, dass** R² in Formel (II) für Methyl steht und die Ketoestergruppen der Formel (II) somit Acetoacetatgruppen sind.

10. Härtbare Zusammensetzung gemäss einem der Ansprüche 1 bis 9, **dadurch gekennzeichnet, dass** die Verbindung mit zwei oder mehr 1,3-Ketoestergruppen der Formel (II) ausgewählt ist aus der Gruppe bestehend aus 1,2-Propandioldiacetoacetat, Dipropylenglykoldiacetoacetat, Tripropylenglykoldiacetoacetat, 1,3-Propandioldiacetoacetat, 1,4-Butandioldiacetoacetat, 2-Methyl-1,3-propandioldiacetoacetat, 1,5-Pentandioldiacetoacetat, Neopentylglykoldiacetoacetat, 1,6-Hexandioldiacetoacetat, 3-Methyl-1,5-pentandioldiacetoacetat, 2-Ethyl-1,3-hexandioldiacetoacetat, Isosorbid-diacetoacetat, 4,4'-Isopropyliden-bis(cyclohexanol)diacetoacetat, Glycerin-diacetoacetat, Glycerin-triacetoacetat, 1,1,1-Trimethylolethandiacetoacetat, 1,1,1-Trimethylolethantriacetoacetat, 1,1,1-Trimethylolpropandiacetoacetat, 1,1,1-Trimethylolpropantriacetoacetat, dem Triacetoacetat von propoxyliertem 1,1,1-Trimethylolpropan mit insgesamt einem mittleren Molekulargewicht Mₙ von 500 bis 2'000 g/mol, Poly(oxy-1,2-propylen)dioldiacetoacetat mit mittlerem Molekulargewicht Mₙ von 600 bis 5'000 g/mol, Poly(oxy-1,2-propylen)trioltriacetoacetat mit mittlerem Molekulargewicht Mₙ von 2'000 bis 6'000 g/mol, Ethylenoxid-Einheiten enthaltendem Poly(oxy-1,2-propylen)triol-triacetoacetat mit mittlerem Molekulargewicht Mₙ von 2'000 bis 6'000 g/mol, Dimerfettsäure-basiertem Polyesterdioldiacetoacetat mit mittlerem Molekulargewicht Mₙ von 1'000 bis 2'500 g/mol, Trimerfettsäure-basiertem Polyestertrioltriacetoacetat mit mittlerem Molekulargewicht Mₙ von 1'000 bis 3'000 g/mol, Rizinusöl-diacetoacetat und Rizinusöl-triacetoacetat.

11. Härtbare Zusammensetzung gemäss einem der Ansprüche 1 bis 10, **dadurch gekennzeichnet, dass** bezogen auf die gesamte Zusammensetzung weniger als 10 Gewichts-%, bevorzugt weniger als 5 Gewichts-%, insbesondere weniger als 1 Gewichts-%, flüchtige organische Lösemittel mit einem Siedepunkt bei Normaldruck von weniger als 250 °C und weniger als 10 Gewichts-%, bevorzugt weniger als 5 Gewichts-%, insbesondere weniger als 2 Gewichts-%, Wasser enthalten sind.

12. Härtbare Zusammensetzung gemäss einem der Ansprüche 1 bis 11, **dadurch gekennzeichnet, dass** die Zusammensetzung als zweikomponentige Zusammensetzung umfassend zwei voneinander getrennt verpackte Komponenten eingesetzt wird, wobei die Polymere mit Endgruppen der Formel (I) ein Bestandteil der ersten Komponente und die Verbindungen mit zwei oder mehr 1,3-Ketoestergruppen der Formel (II) Bestandteil der zweiten Komponente sind.

13. Ausgehärtete Zusammensetzung erhalten aus der härtbaren Zusammensetzung gemäss einem der Ansprüche 1 bis 12 nach dem Vermischen der Inhaltsstoffe beziehungsweise der Komponenten.

14. Ausgehärtete Zusammensetzung gemäss Anspruch 13, **dadurch gekennzeichnet, dass** die Zugfestigkeit mindestens 4 MPa und/oder die Bruchdehnung mindestens 200 %, insbesondere mindestens 300 %, beträgt, bestimmt gemäss DIN EN 53504 bei einer Zuggeschwindigkeit von 200 mm/min an hantelförmigen Prüfkörpern mit einer Dicke von 2 mm und einer Länge von 75 mm bei einer Steglänge von 30 mm und einer Stegbreite von 4 mm.

15. Verwendung der härtbaren Zusammensetzung gemäss einem der Ansprüche 1 bis 12 als elastischer Klebstoff, elastischer Dichtstoff oder elastische Beschichtung, wobei die Inhaltsstoffe beziehungsweise die Komponenten der Zusammensetzung miteinander vermischt werden und die vermischte Zusammensetzung im flüssigen Zustand auf mindestens ein Substrat appliziert wird.
